# EUROPÄISCHE PATENTSCHRIFT

(11) **EP 1 365 839 B1**
(45) Veröffentlichungstag und Bekanntmachung des Hinweises auf die Patenterteilung: **27.08.2008**
(21) Anmeldenummer: 02726054.6
(22) Anmeldetag: 05.03.2002
(51) Int. Cl.: A61N 5/06

(54) **BESTRAHLUNGSANORDNUNG ZUR BEHANDLUNG VON AKNE UND AKNENARBEN**
IRRADIATION ARRANGEMENT FOR THE TREATMENT OF ACNE AND ACNE SCARS
ENSEMBLE D'IRRADIATION DE TRAITEMENT DE L'ACNE ET DES PEAUX ACNEIQUES

(30) Priorität: 08.03.2001 DE 20109899 U; 10.05.2001 DE 10123926
(43) Veröffentlichungstag der Anmeldung: 03.12.2003
(73) Patentinhaber: OptoMed Optomedical Systems GmbH, 12489 Berlin (DE); Spectrometrix Optoelectronic Systems GmbH, 12489 Berlin (DE)
(72) Erfinder: WILKENS, Jan, Henrik, 66242 Homburg (DE); STIRNER, Rolf, 15831 Mahlow-Waldblick (DE)
(74) Vertreter: Patentanwälte Bressel und Partner
(86) Internationale Anmeldenummer: PCT/DE2002/000775
(87) Internationale Veröffentlichungsnummer: WO 2002/072199

(56) Entgegenhaltungen:
- EP-A- 0 726 083
- EP-A- 0 765 674
- EP-B- 0 565 331
- WO-A-00/02491
- WO-A-02/32505
- WO-A-98/23329
- DE-A- 19 838 304
- DE-A- 19 852 524
- US-A- 5 562 706
- US-A- 5 798 523
- US-A- 5 968 034
- US-B1- 6 171 332

## Beschreibung

Die Erfindung betrifft eine Bestrahlungsanordnung zur Behandlung von Akne und Aknenarben.

Es ist bekannt, Akne, eine aufgrund von Bakterienwachstum in verstopften Follikeln talgdrüsenreicher Hautbezirke mit Verhornungsstörungen hervorgerufene Hauterkrankung, mit blauem Licht im Bereich von 400 - 440 nm ohne wesentliche UVA-Anteile zu behandeln, wobei die Erfolge beschränkt blieben. Hierzu sei auf den Fachartikel "V. Sigurdsson et al. Phototherapy of Acne Vulgaris with visible Light, Dermatologie 1997; 194; Bd. 3, 256 -260" mit weiteren Literaturhinweisen verwiesen. Angestoßen wurde diese Form der Therapie, daß Aknefollikel im Rahmen der dermatologischen Untersuchung mit einer sogenannten "woodlamp" rot fluoreszieren. Als Quelle der Fluoreszenz wurde die Speicherung großer Mengen von Porphyrinen im Propionibakterium acne nachgewiesen. (Mc Ginley et al., Facial follicular porphyrin fluorescence. Correlation with age and density of propionibacterium acnes, Br. J. Dermatol Vol. 102., Bd. 3, 437 -441, 1980). Da Porphyrine ihre Hauptabsorption (Soret-band) um 420 nm haben, war es für Meffert et al. naheliegend, bakterielle Aknefollikel mit blauem Licht zu behandeln. Die langwelligste Absorptionsbande der Porphyrine liegt bei 630 nm mit einer Eindringtiefe von 4 mm, die für eine photodynamische Follikelbehandlung am besten geeignet ist und auch verwendet wird.

Aus der WO 00/02491 ist eine derartige Bestrahlungsanordnung bekannt, die mindestens ein schmalbandiges Spektrum im Bereich von 405 - 440 nm umfaßt. Als alternative oder kumulative Spektralbereiche sind die Wellenlängenintervalle von 610 - 670 nm bzw. 520 - 550 nm angegeben. Zur weiteren Verbesserung des Wirkungsgrades wird vorgeschlagen, die zu bestrahlende Partie mit Sauerstoff anzureichern, indem mit Sauerstoff angereicherte Emulsionen vor oder während der Bestrahlung auf die zu bestrahlende Fläche aufgetragen werden. Die Bestrahlungsstärke liegt dabei
zwischen 10 - 500 mW/cm².

Weiter ist aus der WO 00/64537 eine Bestrahlungsanordnung zur Behandlung von Akne bekannt. Dabei wird die zu behandelnde Fläche mit UV-Licht im Bereich von 320 - 350 nm behandelt. Die dabei eingestrahlte Energie wird mit 1 - 5 J/cm² angegeben. Bei Verwendung eines Lasers soll dabei die Pulsenergie zwischen 5 - 25 mJ/cm² liegen, so daß sich bei Pulslängen von 10 ns Bestrahlungsstärken von ca. 2 MW/cm² einstellen. Die bekannte Bestrahlungsanordnung geht dabei von der Erkenntnis aus, daß sonnenähnliche Spektren nicht zur Behandlung von Akne geeignet sind, sondern vielmehr sogar Akneschübe auslösen können.

Aus der EP 0 565 331 B1 ist eine Vorrichtung zur Behandlung von Gefäßerkrankungen in einem Bereich der Haut bekannt, umfassend ein Gehäuse, mit einer inkohärenten Lichtquelle, montiert in dem Gehäuse und geeignet zum Produzieren von gepulstem Licht für die Behandlung und eine Öffnung in dem Gehäuse, welche einen austretenden Lichtstrahl bestimmt, der auf den Hautbehandlungsbereich gesendet wird, ohne durch ein Kabel aus optischen Fasern zu gehen, und der einen breiteren Strahlungsbereich aufweist als Vorrichtungen mit Kabel aus optischen Fasern, wobei die Vorrichtung ein die niedrigen Frequenzen abschneidendes Filter umfaßt, um die sichtbaren und ultravioletten Teile des Spektrums herauszuschneiden und die inkohärente Lichtquelle einen Ausgangslichtstrahl mit Wellenlängen im Bereich zwischen 300 und 1000 nm produziert. Die Lichtquelle ist elektrisch mit einer Variabel-Impulsbreiten-Erzeugerschaltung verbunden, um einen geregelten Zeitimpuls zu liefern mit einer Breite zwischen 1 und 10 ms, wobei der austretende Lichtstrahl auf der Haut eine Energiedichte zwischen 30 und 100 J/cm² erzeugt, so daß der hinaustretende Lichtstrahl nach Durchgang durch das obengenannte, die niedrigen Wellenlängen abschneidende Filter in die Haut so tief wie gewünscht hineindringen kann, ohne die Haut zu verbrennen, um ein unter der Haut und innerhalb des Hautbehandlungsbereiches liegendes Blutgefäß zu erwärmen und im Blutgefäß Blutkoagulation zu verursachen. Die dort beschriebene Blutkoagulation ist bei der Behandlung von Akne zu vermeiden, so daß die dort beschriebene Vorrichtung zur Behandlung von Akne oder anderer oberflächlicher Hauterkrankungen ungeeignet ist.

Aus der DE 93 21 497 U1 ist eine therapeutische Behandlungsvorrichtung bekannt, die mit einer inkohärenten Lichtquelle betrieben wird und Lichtpulse abgibt, wobei vorzugsweise Blitzlichtlampen verwendet werden, die im Wellenlängenbereich von 300-1000 nm emittieren. Auch dort wird die Koagulierung von Blutgefäßen angestrebt. Die dabei angegebenen Energiedichten pro Puls betragen dabei zwischen 30-100 J/cm², sodass auf die Ausführungen zur EP 0 565 331 B1 bezug genommen werden kann.

Immunologische Unterschungsergebnisse haben bei Patienten mit Akne keine abnormen Reaktionen gezeigt, so dass immunoligische Vorgänge beim Zustandekommen der primären Akne vermutlich keine Rolle spielen. Sekundär kommt es z.B. durch Platzen der Epithelien der geschlossenen Komedonen zu einer Verbringung der Komedonenpfröpfe (wie Hornzellen, Haare, Talg, freie Fettsäuren und Bakterien) in das Bindegewebe. Hierdurch entsteht eine Fremdkörperreaktion, die oft durch eine heftige Entzündung in der Aknehaut, der so genannten sekundär-entzündlichen Effloreszenz begleitet wird.

Nach Abklingen der Entzündung entsteht eine dritte Kategorie von Akneeffloreszenzen, die einen früher durchgemachten schweren Akneverlauf charakterisieren und insbesondere kosmetisch stören. Die aknebedingten Narben reichen von kleinen, wie geschlossene Komedone aussehenden Narben über wurmstichartig eingesunkene bis hin zu großflächigen keloidförmigen atrophischen Narben.

Die bekannten Bestrahlungsanordnungen zur Behandlung von Akne sind jeweils technisch sehr aufwendig und damit kostenintensiv, insbesondere wenn in den ausgewählten Spektralbereichen die hohen Energiedichten gefordert werden.

Die bekannten Bestrahlungsanordnungen sind nicht in der Lage, die postentzündlichen Akneeffloreszenzen ohne Gewebekoagulation oder Gewebeabtragung zu behandeln. Somit ist die Therapie von Aknenarben unbefriedigend gelöst. Die Exzision kraterartiger Narbeneinziehungen ist invasiv und mit einem Infektionsrisiko behaftet. Aknenarben im Gesicht können mit hochtourigen Schleifgeräten, keloidförmige Aknenarben oft kryochirurgisch mit flüssigem Stickstoff behandelt werden. Auch diese Verfahren sind sehr aufwendig und kostenintensiv.

Aus der EP 0 726 083 A2 ist eine Vorrichtung zur Diagnose und Behandlung von Krebs bekannt, wobei die breitbandige Bestrahlungsquelle Filter aufweist, wobei im Diagnosemodus ein Spektrum von 350-500 nm emittiert wird mit einem Peak bei 400 nm. Die Bestrahlungsquelle kann dabei entweder gepulst oder cw betrieben werden. Im Puls-Mode ist die Pulsfrequenz bei 0,02 bis 2 Pulsen/Sekunde. Die Pulsdauer liegt im Bereich von 0,1-1000 nm und die Energiedichte bei 0,02-4 J/cm² pro Puls.

Aus der US - 5,968,034 ist eine im Pulsbetrieb betreibbare breitbandige Bestrahlungsquelle bekannt, die bevorzugt im Bereich von 700-1800 nm emtittiert.

Aus der DE 198 38 304 A1 ist ein Externum oder objektnah zu installlierendes, leuchtstoffhaltiges Mittel für phototherapeutische Zwecke für den Einsatz beim Menschen, beim Tier und bei den Pflanzen bekannt, wobei in eine geeignete Grundlage ein für den entsprechenden Zweck geeigneter Leuchtstoff eingearbeitet wird, welcher direkt auf die zu behandelnde Stelle aufgetragen und danach mit definierten Dosen einer UVC-Strahlung bestrahlt wird oder eine leuchtstoffhaltige Folie oder Filter im Lichtaustrittselement der UVC-Strahlen reversibel integriert wird.

Aus der WO 98/23329 A1 ist eine breitbandige cw-Bestrahlungsquelle bekannt, die zwischen 360-3000 nm emittiert, wobei die Bestrahlungsstärke vorzugsweise unter 800 mW/cm² ist.

Aus der US - 5,562,706 ist eine Vorrichtung zur Erzeugung eines dynamischen magnetischen Feldes bekannt, dass auch optische und elektrische Pulse erzeugen kann.

Aus der EP 0 765 674 A2 ist eine breitbandige, pulsbare Bestrahlungsquelle zur Behandlung von Psoriasis bekannt, die im Bereich von 400-1000 nm emittiert, wobei vorzugsweise im längerwelligen Bereich emittiert wird.

Aus der US - 6,171,332 B1 ist ein Verfahren zur Behandlung unter anderem von Psoriasis oder Entfernung von Tattoos bekannt, wobei eine nichtkohärente, schmalbandige Bestrahlung, vorzugsweise mit einer Bandbreite von 30 nm, Verwendung findet. Die bevorzugte Wellenlänge liegt bei 620 nm, wobei die Bestrahlung auch in Form von Pulsen abgegeben werden kann. Die Bestrahlungsstärke liegt über 0,075 W/cm².

Aus der DE 198 52 524 A1 ist eine Bestrahlungseinrichtung für therapeutische und kosmetische Zwecke bekannt, umfassend mindestens eine optische Strahlungsquelle, die auf einer zu bestrahlenden Fläche im Wellenlängenintervall von 400-440 nm eine Bestrahlungsstärke von mindestens 20 mW/cm² erzeugt und im Wellenlängenintervall von 340-400 nm eine Bestrahlungsstärke von weniger als 2 mW/cm² erzeugt.

Aus der US - 5,798,523 ist ein Scanner für phototherapeutische Zwecke bekannt.

Der Erfindung liegt daher das technische Problem zugrunde, eine Bestrahlungsanordnung und ein Verfahren zur Behandlung von Akne und Aknenarben zu schaffen, die kostengünstig realisierbar sind und einen hohen Wirkungsgrad aufweisen.

Die Lösung des technischen Problems ergibt sich durch den Gegenstand mit den Merkmalen des Patentanspruchs 1. Weitere vorteilhafte Ausgestaltungen der Erfindung ergeben sich aus den Unteransprüchen.

Hierzu ist die Bestrahlungsquelle als breitbandige Bestrahlungsquelle mit einem Wellenlängenbereich von mindestens 320 bis mindestens 540 nm ausgebildet, die zur Erzeugung von optischen Pulsen im Pulsbetrieb betreibbar und/oder relativ zur Behandlungsfläche durch einen Scanner bewegbar ist, wobei, wenn die Bestrahlungsanordnung auf die Oberfläche aufgesetzt würde, die Pulsenergie pro Puls zwischen 0,05 - 10 J/cm² und die Bestrahlungsstärke der Bestrahlungspeaks der optischen Pulse zwischen 0,5 W/cm² und 100 kW/cm² liegt. Mindestens 320 nm bedeutet dabei, daß die Bestrahlungsquelle durchaus auch kleinere Wellenlängen erzeugen kann, diese aber nicht auf die zu behandelnde Fläche weitergeleitet werden, sondern vorher unterdrückt werden. Größere Wellenlängen als 540 nm hingegen können mitabgestrahlt werden. Erfindungsgemäß wird ausgenutzt, daß entgegen Ausführungen in der Literatur, durch den Pulsbetrieb bzw. Pulse die Bildung von Singulett-Sauerstoff um Größenordnungen gegenüber cw-Betrieb bei gleicher Energie erhöht wird. Dabei ergibt sich überraschenderweise, daß durch die Verwendung von Pulsen die mittlere spektrale Bestrahlungsstärke unter die aus der Literatur bekannten Schwellwerte gedrückt werden kann, obwohl die Wirksamkeit erhöht wird. Da entgegen den Ausführungen in der WO 00/64537 auch sichtbare Spektralbereiche behandlungswirksam sind, kann auf preiswerte breitbandige Bestrahlungsquellen zurückgegriffen werden, so daß kostenintensive Laser oder Filtermaßnahmen entbehrlich sind. Die Ursache für die Akneschübe mit solarähnlichen Strahlungsquellen liegt nämlich vermutlich nicht im sichtbaren Anteil, sondern in dem UVB-Anteil bis 320 nm, auf den erfindungsgemäß auch nicht zurückgegriffen wird. Ein weiterer Vorteil gegenüber der WO 00/02491 ist, daß eine größere Leistung bzw.Energie des blauen Spektralanteils zum Follikel gelangt. Da die Follikel relativ tief unter der Haut sitzen, erreicht normalerweise nur ein Bruchteil des blauen Spektralbereichs zwischen 400 - 500 die Follikel, so daß Bestrahlungsanordnungen mit einem reinen Blauspektrum mit relativ hoher Leistung arbeiten müssen. Dies liegt zum einen an der geringen Eindringtiefe des blauen Spektralanteils und zum anderen vermutlich an Schwelldosen aufgrund von körpereigenen Antioxidantien. Hierin ist auch eine mögliche Erklärung für die bescheidenen Ergebnisse von Sigurdsson zu sehen, wo nur mit geringen Bestrahlungsstärken unterhalb oder im Bereich dieser Schwellwerte gearbeitet wurde. Überraschenderweise kann erfindungsgemäß mit mittleren Bestrahlungsstärken ca. 1 Größenordnung unterhalb der in der WO00/02491 beschriebenen Schwellwerte von 60 mW/cm² und unterhalb der verwendeten Flächenleistungen von Sirgudsson und Meffert eine gute Wirksamkeit erzielt werden, wenn die Spitzenleistung bzw. Bestrahlungsstärke des Pulses nur kurze Zeit die beschriebenen Schwellwerte deutlich überschreiten. Klinische Untersuchungen zeigen, daß die Effizienz einer gepulsten Aknelichtbehandlung im Vergleich zur cw-Behandlung bei identischem Spektrum überraschend um den Faktor 10 bis 20 gesteigert werden kann. Die beschriebene Wirkung für die Aknebehandlung in der WO 00/02491 liegt wahrscheinlich in einer oberflächlichen Bakterienabtötung durch den Blauanteil, wohingegen die Follikel im wesentlichen nur durch die grünen bzw. roten Spektralbereiche von 520 - 550 nm bzw. 610 - 670 nm erreicht werden. Durch den Pulsbetrieb wird hingegen bei gleicher cw-Leistung temporär mit extrem höheren Leistungen gegenüber cw-Betrieb bestrahlt, so daß ein konstanter off-set aufgrund von Schwellwerten weniger stark ins Gewicht fällt. Somit erreicht effektiv mehr blaues Licht das Follikel und kann zur Bildung von Singulett-Sauerstoff beitragen. Erfindungsgemäß können nicht nur die entzündlichen Akneeffloreszenzen, sondern auch die kosmetisch störenden Aknenarben mit sehr guten Resultaten behandelt werden, da es überraschenderweise zu einer Entfärbung der oft pigmentierten Narben mit einer gleichzeitigen Verflachung der Narbe kommt. Im Ergebnis wird durch die Bestrahlungsanordnung nicht nur die akute Entzündung unterdrückt, sondern auch bereits bestehende Aknenarben werden erheblich verkleinert. Dies geschieht insbesondere dadurch, dass das eingestrahlte Licht über die Entstehung von Singulettsauerstoff in der Aknenarbe eingeschlossene Pigmente ausbleicht und es vermutlich radikal vermittelt zu einem feingeweblichen Umbau der Haut kommt, der die Zeichen der Hautalterung, Falten, epidermale und dermale Atrophie, Hautrauhigkeit, unregelmäßige Pigmentation, Telangiektasien, Schlaffheit der Haut und vergrößerte Poren bessert bzw. beseitigt. Das jüngere Hauterscheinungsbild geht auf eine durch die Bestrahlungsanordnung ausgelöste dermale Stoffwechseländerung, insbesondere im Bereich der extrazellulären dermalen Matrixproteine einher. Es kommt zu einer Zunahme von Prokollagen, Kollagen, Elastin und Kollagenasen als Zeichen eines durch die Bestrahlung angestossenen Remodelings der Hautstruktur. Im Ergebnis bilden sich die die kosmetisch störenden Hautveränderungen deutlich oder sogar vollständig zurück.

Weiter ist der Bestrahlungsanordnung eine Einrichtung zur Erzeugung von mechanischen Schwingungen zugeordnet, wobei vorzugsweise die mechanischen Schwingungen zeitlich versetzt zu den optischen Pulsen erzeugt werden. Dabei wird der teilweise bereits sehr feste Talg durch die optischen Pulse erwärmt und verflüssigt. Durch die anschließende mechanische Schwingung wird dann der verflüssigte Talg aus der Pore quasi herausgerüttelt.

In einer bevorzugten Ausführungsform ist die Einrichtung als elektrodynamischer Transducer ausgebildet. Dabei wird vorzugsweise durch geeignete Polung von gegenüberliegenden Flachspulen jeweils eine Druck- bzw. Zugkraft auf die Hautoberfläche ausgeübt, wobei zur Ansteuerung der Spulen der Strom der Bestrahlungsquelle verwendet werden kann. Alternativ kann die Einrichtung auch als photoelastischer Transducer ausgebildet sein, wo die sprunghafte Ausdehnung eines Materials durch den optischen Puls zur Erzeugung der mechanischen Schwingung ausgenutzt wird.

Die Angaben zur Leistungs- bzw. Energiedichte beziehen sich dabei immer auf die zu bestrahlende Hautoberfläche, wobei vorzugsweise die Bestrahlungsanordnung direkt auf die Haut aufgesetzt wird.

Vorzugsweise liegen die effektiven Pulslängen zwischen 1 *µ*s und 500 ms.

Dieser relativ breite Bereich kommt daher, daß die bevorzugten effektiven Pulslängen für gepulste Bestrahlungsquellen und relativ bewegte Bestrahlungsquellen in Form eines Scanners unterschiedlich sind. Dabei ist anzumerken, daß der Scanner bevorzugt zur Behandlung größflächiger Akne zur Anwendung kommt.

Bei den Blitzlampen liegen die effektiven Pulslängen vorzugsweise zwischen 1µs und 50 ms, besonders bevorzugt zwischen 10 µs und 10 ms und weiter bevorzugt zwischen 100-600 µs, wobei die Pulsein- und auszeiten unsymmetrisch sind.

Bei einer Ausbildung als Scanner liegen die effektiven Pulslängen vorzugsweise zwischen 1 ms und 500 ms, weiter vorzugsweise zwischen 20-100 ms. Unter effektiver Pulslänge wird dabei die Zeit verstanden, die zwischen Erreichen von 50 % der maximalen Leistung bis zum Abfall auf 50% der maximalen Leistung liegt. Die im Verhältnis zur effektiven Pulslänge längeren Pulsauszeiten dienen dabei insbesondere der Nachdiffusion von Sauerstoff. Das Verhältnis Pulslänge und Pulsauszeit liegt dabei vorzugsweise beim Scanner zwischen 3 und 3000 und bei der Blitzlampe zwischen 100 und 100.000.

In einer weiteren bevorzugten Ausführungsform liegt die Frequenz, mit der die Bestrahlungsquelle gepulst wird, zwischen 0,01 - 100 Hz, weiter bevorzugt zwischen 0,05-50 Hz und noch bevorzugter zwischen 0,3-3 Hz, wobei bei höheren Frequenzen niedrigere effektive Pulslängen und kleinere Pulsenergien verwendet werden.

Ebenso wie die effektiven Pulslängen davon abhängig sind, ob eine gepulste Bestrahlungsquelle oder eine relativ bewegte Bestrahlungsquelle verwendet wird, sind auch die bevorzugten Bestrahlungsstärken bzw. Leistungsdichten pro Puls unterschiedlich.

Bei Ausführungsformen mit gepulster Bestrahlungsquelle liegt die Bestrahlungsstärke pro Puls zwischen 1 W/cm²-100 kW/cm², vorzugsweise zwischen 50 W/cm²-50 kW/cm², weiter bevorzugt zwischen 500 W/cm²- 10 kW/cm² und besonders bevorzugt zwischen 1 kW/cm²-5 kW/cm². Die Energiedichte pro Puls liegt dabei zwischen 50 mJ/cm²-10J/cm², vorzugsweise zwischen 100 mJ/cm²-2J/cm² und besonders bevorzugt zwischen 300-1000 mJ/cm².

Bei Ausführungsformen mit einem Scanner, wobei zusätzlich die Bestrahlungsquelle auch gepulst sein kann, liegen die Bestrahlungsstärken pro Puls zwischen 500 mW/cm²-5000W/cm², vorzugsweise zwischen 1-500 W/cm² und besonders bevorzugt zwischen 2-300 W/cm² und noch bevorzugter zwischen 3-100 W/cm². Die Energiedichte pro Puls liegt dabei zwischen 50 mJ/cm²-10J/cm², vorzugsweise zwischen 100-3000 mJ/cm² und besonders bevorzugt zwischen 150-1000 mJ/cm² und noch bevorzugter zwischen 200-500 mJ/cm².

Die Erzeugung längerer effektiver Pulslängen ist mit den bekannten Blitzlampen kaum oder gamicht realisierbar. Dies kann jedoch zur selektiven Anwärmung des Talgfollikelbereiches und des Haarschafts vorteilhaft sein, um eine Verflüssigung der obstruierenden Talgkonkremente und eine induzierte Störung der Talgproduktion zu erreichen. Ein weiterer positiver Effekt könnte eine Verminderung der Verhornung bzw. Abschilferung von Epithelzellen im Haarschaftsbereich sein. Derartige längere Pulslängen lassen sich jedoch in ihrer thermokinetischen Wirkung durch eine gezielte Ablaufsteuerung simulieren. Hierzu werden beispielsweise 100 Pulse mit einer effektiven Pulslänge von 100 µs und einer Pulsauszeit von 900 µs eingestrahlt, wobei anschließend für 10-1000 ms keine Pulse folgen. Anschließend werden dann wieder 100 Pulse eingestrahlt. Die hierbei verwendeten effektiven Pulslängen liegen zwischen 50-300 µs.

In einer weiteren bevorzugten Ausführungsform ist die Bestrahlungsquelle als Xe-Blitzlampe ausgebildet. Diese handelsüblichen Xe-Blitzlampen sind sehr preiswert und emittieren ausreichend im gewünschten Spektralbereich zwischen 320 -540 nm bzw. 320-670 nm. Hierzu wird beispielsweise auf die US 4.167.669 oder die EP 0 565 331 verwiesen, wobei die dort beschriebenen Pulsenergien für die erfindungsgemäße Lehre jedoch zu groß sind. Xe-Blitzlampen sind je nach Belastung mehr oder weniger vom Spektrum mit einem Schwarzen Körper vergleichbar. Daher emittieren Xe-Blitzlampen typischerweise von 200-2000 nm. Aufgrund der Zelltoxizität der Wellenlängen zwischen 200-320 nm muß dieser Wellenlängenbereich durch entsprechende Filtermaßnahmen unterdrückt werden. Des weiteren ist es möglich, die Xe-Blitzlampe mit Metallen oder Metallhalogeniden zu dotieren, um gezielt die Emission bestimmter Spektralanteile zu verstärken. Besonders geeignet hierfür sind Gallium, Indium und/oder deren Halogenide, um den Wellenlängenbereich zwischen 400-500 nm zu verstärken.

In einer weiteren bevorzugten Ausführungsform ist der Bestrahlungsquelle eine Einrichtung zur Unterdrückung der Spektralanteile von 320 - 400 nm und/oder zur Transformierung der UV-Anteile in den sichtbaren Bereich zugeordnet. Hiermit wird dem Umstand Rechnung getragen, daß die möglichen Nebenwirkungen von UVA-Anteilen auf die Zelle gänzlich vermieden werden, ohne daß sich dies in einer spürbaren Reduzierung der Wirksamkeit der Bestrahlungsanordnung niederschlägt. Werden die UVA-Anteile herausgefiltert, so können preiswerte handelsübliche UVA-Filter zur Anwendung kommen. Vorzugsweise werden jedoch die UV-Anteile mittels geeigneter anorganischer Leuchtstoffe und organischer Laserfarbstoffe in den sichtbaren Spektralbereich transformiert. Dabei haben sich besonders aus Silikonelastomeren bestehende Folien mit anorganischen Leuchtstoffen bewährt. Aufgrund der hohen Absorption der Porphyrine um 420 nm werden bevorzugt blau emittierende Leuchtstoffe verwendet, die gegebenenfalls mit grünen im Bereich von 520 - 550 nm und/oder roten im Bereich 610 - 670 nm kombiniert werden können. Alternativ können statt der Silikonelastmere auch Fluorpolymere wie beispielsweise Teflon zur Anwendung kommen. Bei der Verwendung von Leuchtstoffen zur Transformierung der unerwünschten Spektralanteile können auch andere Bestrahlungsquellen wie beispielsweise Deuterium-Blitzlampen zur Anwendung kommen, die eine hohe Ausbeute im UV-Bereich aufweisen, der dann durch die Leuchtstoffe in den gewünschten Spektralbereich transformiert werden kann.

Eine weitere mögliche Bestrahlungsquelle ist eine im Überlastbereich gepulste galliumjodid-Quecksilberentladungslampe. Dabei wird unter Überlast verstanden, wenn der maximale Entladungsstrom mindestens 3-1000 fach über dem Lampennennstrom liegt, wobei der Impulsentladungsstrom vorzugsweise zwischen 15-1500 A/cm² Querschnittsfläche des Entladungsgefäßes liegt. Handelsübliche cw-betriebene metalldampfdotierte Quecksilberhalogenid-Lampen sind beispielsweise in der US-3,521,111; US-3,540,789 und der WO 96/13851 beschrieben.

Aus der US 5,184,044 ist zu entnehmen, daß unter Berücksichtigung der Lampengeometrie, der Lampenleistung von 20 W und dem Spannungsabfall von 55 V ein Lampenstrom von 8 A/cm² Querschnittsfläche des Entladungsgefäßes der maximal sinnvollen Lampenbelastung entspricht, da es bereits zu einer Inversion im Bereich der Indiumresonanz kommt. Eine weitere Erhöhung der Stromdichte würde die Inversion bis zur Löschung verstärken.

Deshalb wurden diese bisher nicht im Überlastbetrieb gefahren, da es aus plasmaphysikalischen Untersuchungen bekannt war, dass schmalbandige Emissionsspektren nur bei vergleichsweise niedriger Anregungsenergie darstellbar sind. Im Überlastbetrieb steigt der Dampfdruck im Entladungslasma so stark an, dass die Linienemission vom Umgebungsplasma absorbiert wird und es paradoxerweise zu einer erheblichen Schwächung oder sogar einer kompletten Löschung der Resonanzlinienemission bei hohen Entladungsdrücken kommt. Als Beispiele seien die Quecksilberresonanzlinie bei 254 nm, die Natriumresonanzlinie bei 488 nm und die Indiumresonanzlinie bei 450 nm genannt, wo es schon bei mäßiger Überlast zu einem Verlust der Spektralintensität kommt.

Überraschend wurde jedoch entdeckt, daß galliumdotierte Quecksilberjodid-Mittel- bzw. Hochdrucklampen selbst bei einer 100-1000 fachen Überlast weder eine Verbreiterung oder gar Inversion der Galliumresonanzlinien bei 403 und 417 nm zeigen. Eine unter Nennlast mit einem Entladungsstrom von 1,5 A/cm² Querschnittsfläche des Entladungsgefäßes betriebene galliumjodiddotierte Quecksilberentladungslampe konnte im Pulsbetrieb bis auf 1000 A/cm² Querschnittsfläche des Entladungsgefäßes betrieben werden, ohne daß es zu einer Abschwächung oder Frequenzinversion der Galliumresonanzlinien kam. Eine mögliche Erklärung hierfür ist, daß das metallische Gallium einen Siedepunkt von ca. 2200°C aufweist, so daß der diesbezügliche Galliumdampfdruck auch unter Impulsbedingungen vermutlich zu vernachlässigen ist. Im Plasma kommt es zu einer Zersetzung des Quecksilberiodids in Quecksilber und Jod. Das Jod verbindet sich unter den Entladungsbedingungen mit dem Gallium in Form der instabilen Verbindung Gal₃. Galliumiodid zeigt eine starke Dampfdruckzunahme bereits bei niedrigen Temperaturen. Die fehlende Inversion der Galliumresonanzlinie wäre nun dadurch erklärbar, dass das Galliumiodid sich nur bis zu einem bestimmten Druck stabil verhält und es bei einer Druckzunahme zu einem massiven, vermutlich kinetisch außerordentlich schnellem Zerfall der Verbindung kommt. In der Folge stellt sich trotz zunehmender hoher Temperaturen während des Impulses ein relativ konstanter Galliumdampfdruck ein. Somit trägt das kondensierte Gallium nicht zur Entladung bzw. zur möglichen Selbstabsorption bei. Der unerwartet aufgetretene Effekt könnte deshalb mit einer paradoxen Konstanz des Galliumdampfdrucks über einen Temperaturbereich von 200 bis fast 2200° C zusammenhängen. Quecksilberjodid zerfällt bereits früh in Quecksilber und Jod, so dass das Jod für eine Galliumverbindung zur Verfügung steht und der Quecksilberdampfdruck mit der eingespeisten Energie stark zunimmt. Auf diese Weise steht der Galliumresonanz entsprechend dem Wirkungsquerschnitt des entsprechenden Galliumplasmas eine proportional zur eingespeisten Energie zunehmende Anregung durch die kurzwelligen Quecksilberelektronenübergänge zur Verfügung. Diese Energie kann wegen der relativen Konstanz des Galliumdampfdruckes fast in der gesamten Höhe als Resonanzlinienspektrum emittiert werden.

Während des Überlastbetriebes kommt es zu einer kurzzeitigen Überhitzung, insbesondere der Wolframwendeln, die jedoch bei einer Zunahme der Temperatur entsprechend dem Planckschen Gesetz erheblich mehr Wärme abstrahlen können. Daher kann eine modulierte Lampe mit einer erhöhten Grundlast betrieben werden, da die Abgabe der zugeführten Energie nur aufgrund der Temperaturerhöhung erheblich effizienter ist als bei einer Lampe im Normalbetrieb. Dabei hat sich herausgestellt, dass sich eine 1 kW Lampe mit einer Dauerlast von ca. 2-20 kW betreiben läßt. Bei spektralen Messungen hat sich gezeigt, daß bei einer 1000 W cw-Betrieb galliumdotierten Quecksilberiodid-Lampe ca. 400 mW/m² im Spektralbereich zwischen 400-440 nm die Haut treffen. Diese Bestrahlungsstärke läßt sich im Simmerbetrieb auf eine gemittelte Bestrahlungsstärke von ca.2-4 mW/cm² vermindern, wobei unter Impulslast die Bestrahlungsstärke kurzzeitig um bis zu vier bis fünf Größenordnungen angehoben wird, so daß Bestrahlungsstärken zwischen 2 bis 400 W/cm² auf die Haut treffen. Das Verhältnis der Pulslängen liegt vorzugsweise im Bereich zwischen 3 und 300. Diese einfache Impulslichtquelle im Spektralbereich von 320-540 nm ist auch für andere technische Anwendungen verwendbar, wie beispielsweise dentale Kunststoffhärtung, drucktechnische Anwendungen, Oberflächenversiegelungen, Rohrsanierung mit lichthärtenden Schläuchen, Kunststoffhärtung im Bereich der DVD-Produktion sowie die Beschleunigung anderer photochemischer Reaktionen, die über Radikalmechanismen einer Photoabsorption im UV-Blaubereich des Spektrums beeinflußt werden.

Das Verhältnis Gallium bzw. Galliumadditiv zu Quecksilber sollte vorzugsweise zwischen 1:10 bis 1:100 sein. Im Leistungsbereich von 400 W kommt vorzugsweise ein Mischungsverhältnis von 1-5 mg Galliumiodid auf 44 mg Quecksilber zur Anwendung.

Eine andere typische Lampe besteht aus einem zylindrischen Quarzrohr mit Durchmesser 13,5 mm und einem Volumen des Entladungsgefäßes von 20 cm³. Der Elektrodenabstand beträgt ca.14 cm. Eine derartige Lampe wird mit 20 mg Hg, 3 mg Quecksilberiodid, 1 mg Gallium und Argon mit einem Druck von 3,57 mmHg befüllt.

Die Effizienz der Bestrahlungsanordnung kann weiter durch eine Erhöhung der Sauerstoffkonzentration gesteigert werden. Neben den in der WO 00/02491 beschriebenen Maßnahmen kann dies auch sehr einfach durch eine inspiratorische Sauerstoffzufuhr über eine Sauerstoffmaske erreicht werden.

Aufgrund der eingestrahlten Pulsenergien kommt es zu einer spürbaren Erhöhung der Hauttemperatur, so daß vorzugsweise eine Kühleinrichtung für die zu bestrahlende Fläche vorgesehen ist. Diese kann im einfachsten Fall als Luftkühlung ausgebildet sein. Ebenso kann der Bestrahlungsquelle eine Kühleinrichtung zugeordnet sein, die beispielsweise als Luftkühlung oder eine andere thermische Ableitmaßnahme wie Kühlbleche ausgebildet ist. Des weiteren wird vorzugsweise auch die Leuchtstofffolie gekühlt, wobei diese jedoch bevorzugt über eine Wasserkühlung gekühlt wird.

Zur Erhöhung der abgestrahlten Leistung in Richtung der zu behandelnden Fläche wird die Strahlungsquelle vorzugsweise mit einem Reflektor ausgebildet. Ein bevorzugter Reflektortyp ist ein Paraboloid-Reflektor, wobei die Bestrahlungsquelle in einem Brennpunkt des Paraboloids angeordnet wird. Prinzipiell sind jedoch auch kugelschalenförmige oder ähnlich geformte Reflektoren verwendbar.

Die Bestrahlungsfläche für ein mobiles Gerät liegt vorzugsweise im Bereich von 1 - 200 cm², da bei flächiger Bestrahlung die Eindringtiefe im Vergleich zu punktförmigen Bestrahlungsquellen zunimmt, was hier zum Erreichen der tiefer liegenden Follikel vorteilhaft ist. Die mobile Ausführungsform erlaubt die sequentielle Bestrahlung verschiedener einzelner Akneherde, die in der Regel im Gesichtsbereich, im Halsbereich sowie im oberen Bereich des Rückens und des Brustkorbes auftreten.

Alternativ sind auch Ausführungsformen möglich, bei denen simultan größere Flächen bestrahlt werden. Eine mögliche Ausführungsform besteht darin, eine Vielzahl kleiner Blitzlampen, beispielsweise 30-60 kleine Xe-Blitzlampen in ein Gewebe einzunähen. Dieses besteht dabei beispielsweise aus PTFE oder einem PTFE-Derivat. Ein derartiges Gewebe kann durch direkte Metallbedampfung hochreflektiv beschichtet werden, wobei die erwünschte Luftdurchlässigkeit bei gleichzeitiger Abweisung von Wasser erhalten bleibt. Bei Verwendung einer Vielzahl von kleinen Strahlungsquellen in räumlicher Nähe zum Bestrahlungsobjekt kann auf die Verwendung eines abbildenden Reflektors verzichtet werden. Mit Hilfe von weichen, strahlungstransparenten Abstandshaltern wie beispielsweise Silikonelastomeren ist eine Kühlung und Hinterlüftung der Filter, Leuchtstofffolien sowie der bestrahlten Hautflächen einfach über handelsübliche Lüfter wie beispielsweise CPU-Lüfter möglich.

Die Erfindung wird nachfolgend anhand eines bevorzugten Ausführungsbeispieles näher erläutert. Die Fig. zeigen:

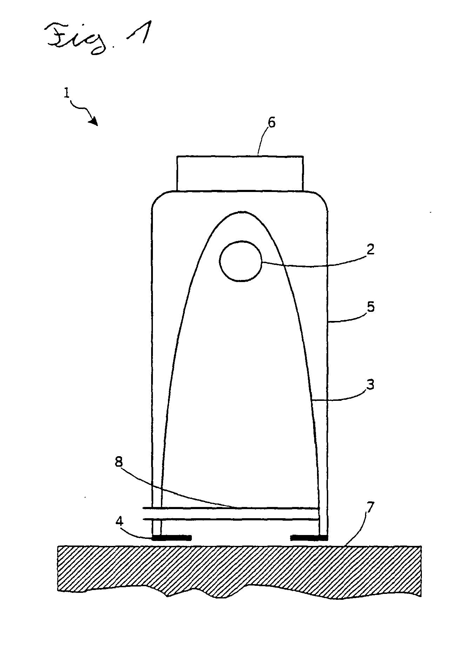

- Fig. 1: einen Querschnitt durch eine Bestrahlungsanordnung,
- Fig. 2: ein Spektrum der Bestrahlungsquelle mit und ohne Leuchtstofffolie, wobei die relative Bestrahlungsstärke über der Wellenlänge dargestellt ist,
- Fig. 3: eine Haut-Querschnittsdarstellung mit Aknefollikel,
- Fig. 4: eine Prinzipdarstellung eines elektrodynamischen Transducers,
- Fig. 5: eine Prinzipdarstellung eines photoelastischen Transducers,
- Fig.6: Verläufe der relativen Bestrahlungsstärke einer galliumdotierten Quecksilberiodid-Lampe in cw-Betrieb und gepulstem Überlastbetrieb,
- Fig.7: die spektrale Energiedichte einer galliumdotierten Quecksilberiodid-Lampe bei verschiedenen Eingangsleistungen,
- Fig.8: Verläufe der relativen Bestrahlungsstärke einer Natriumdampflampe im cw- und Puls-Überlastbetrieb,
- Fig.9: eine schematische Schaltungsanordnung zum Impulsbetrieb einer galliumdotierten Quecksilberiodid-Lampe mit zwei Phasen eines Drehstromnetzes und
- Fig.10: eine alternative Schaltungsanordnung mit einer Kondensatorbank.

Die Bestrahlungsanordnung 1 umfaßt eine breitbandige Bestrahlungsquelle 2, die vorzugsweise als Xe-Blitzlampe ausgebildet ist. Die Bestrahlungsquelle 2 ist in einem Brennpunkt eines Paraboloid-Reflektors 3 angeordnet, der an der dem Brennpunkt abgewandten Seite offen ist. Die Austrittsfläche am offenen Ende des Paraboloidreflektors 3 wird durch eine vorzugsweise verstellbare Blende 4 definiert. Durch die verstellbare Blende 4 kann somit die Größe der zu bestrahlenden Fläche angepaßt werden. Die Bestrahlungsquelle 2 und der Paraboloid-Reflektor 3 sind in einem Gehäuse 5 angeordnet. Das Gehäuse 5 ist vorzugsweise mit einem Handstück 6 ausgebildet, mittels dessen die Bestrahlungsanordnung 1 einfach auf eine zu behandelnde Fläche 7 aufsetzbar ist. Zwischen der Bestrahlungsquelle 2 und der zu behandelnden Fläche 7 ist eine Leuchtstofffolien 8 angeordnet, die mit Leuchtstoffpartikeln dotiert ist. Die Leuchtstofffolie 8 kann auch unmittelbar im Bereich der Bestrahlungsquelle 2 oder aber über die Blende 4 gespannt sein. Vorzugsweise ist die Leuchtstofffolie 8 derart angeordnet, daß diese leicht auswechselbar ist. Dies vereinfacht den aufgrund von Alterungsprozessen notwendigen Austausch, aber auch den flexiblen Einsatz von Leuchtstofffolien mit unterschiedlichen Leuchtstoffpartikeln. Des weiteren kann bei äußerer Anordnung der Leuchtstofffolie 8 diese leicht desinfiziert werden. Die elektrischen Anschlüsse und die Schaltung zur Erzeugung der variablen Pulsbreiten sind hier aus Übersichtsgründen nicht dargestellt.

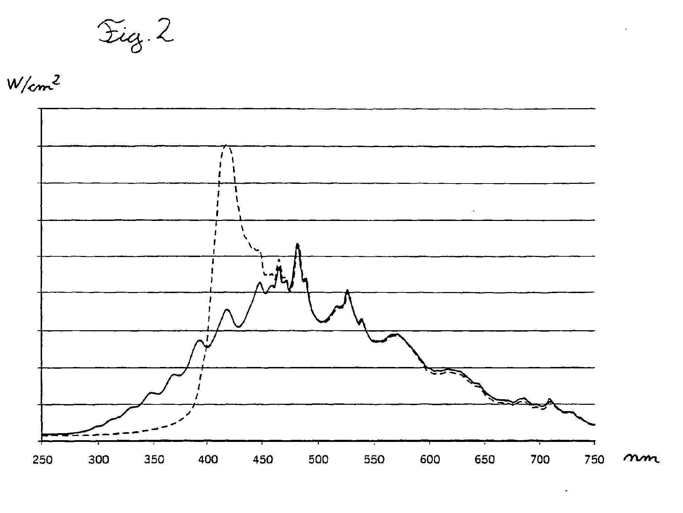

In der Fig. 2 ist ein Spektrum einer verwendeten Xe-Blitzlampe mit und ohne Leuchtstoffolie dargestellt. Das Spektrum mit Leuchtstofffolie ist dabei gestrichelt dargestellt. Bei der Leuchtstoffolie handelt es sich um ein Silikonelastomer, das mit anorganischen Leuchtstoffen dotiert ist, die im blauen Spektralbereich von 400 - 450 nm bevorzugt emittieren. Die Leuchtstofffolie schneidet dabei den UV-Bereich zwischen 280 - 400 nm nahezu ab und transformiert diesen in den sichtbaren blauen Bereich von 400 - 450 nm. Der verbleibende NIR-Anteil ist hier nicht dargestellt.

Die Xe-Blitzlampe wird mit einer Frequenz zwischen 0,01 - 100 Hz, bevorzugt zwischen 0,1 und 10 Hz getaktet, wobei jedoch die effektiven Pulslängen nur zwischen 10 µs und 10 ms liegen. Die optischen Pulsenergien betragen dabei zwischen 0,5 - 10 J/cm², bevorzugt zwischen 1-3 J/cm².

Die Aknebahandlung erfolgt dabei über mehrere Tage bzw. Wochen, wobei die tägliche Behandlungsdauer zwischen 1 und 60 Minuten , vorzugsweise zwischen 5-10 Minuten liegt.

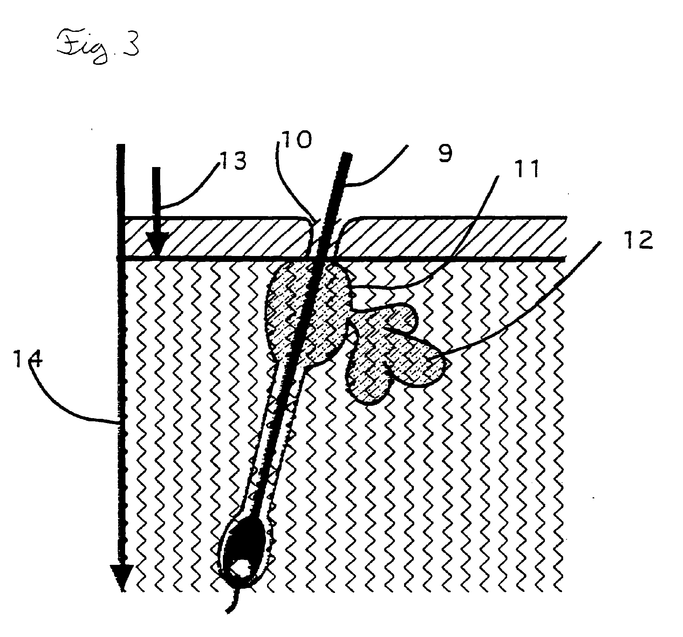

In der Fig.3 ist ein Querschnitt durch die Haut im Bereich eines Haares 9 dargestellt. Das Haar 9 ist über einen verengten Ausführungsgang 10 mit einem mit Talg überfüllten und entzündeten Haarschaftsbereich 11 mit einer vergrößerten und entzündeten Talgdrüse 12 verbunden. Bei einem cw-Betrieb mit blauem Licht wird aufgrund der geringen Eindringtiefe (1/e) des blauen Lichtes sowie der Überwindung körpereigener Schwellwerte für blaues Licht bereits der überwiegende Anteil in der oberen Hautschicht oberhalb des Haarschaftsbereiches absorbiert, was schematisch durch den kurzen Pfeil 13 dargestellt ist. Beim Pulsbetrieb hingegen ist bei gleicher mittlerer Leistungsdichte die Leistungsdichte während des Pulses entsprechend dem Verhältnis der effektiven Pulslänge zur Frequenz wesentlich größer, so daß die konstante Abschwächung aufgrund der körpereigenen Schwellwerte geringer ins Gewicht fällt. Da somit die zur Verfügung stehende effektive Leistung größer ist, erreicht auch ein größerer absoluter Anteil des blauen Lichtes die tieferliegenden Haarschaftsbereiche 11 bzw. die Talgdrüse 12 und können dort zur lokalen Erzeugung von Singulett-Sauerstoff beitragen, was durch den längeren Pfeil 14 dargestellt ist.

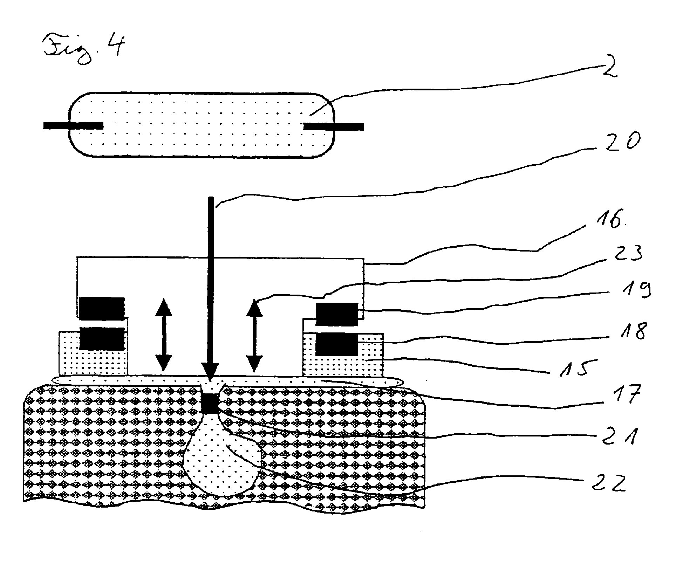

In der Fig.4 ist schematisch eine Prinzipdarstellung eines elektrodynamischen Transducers dargestellt. Die Einrichtung zur Erzeugung von mechanischen Schwingungen umfaßt einen Rahmen 15 und einen transparenten, nicht kompessiblen Stempel 16, der in dem Rahmen 15 beweglich gelagert ist, wobei der Stempel 16 teilweise über ein Ultraschall-Gel 17 als Koppelmedium auf der Haut aufliegt. An den Randbereichen des Rahmens 15 bzw. Stempels 16 sind Flachspulen 18, 19 angeordnet, die sich jeweils gegenüberliegen. Die Flachspulen sind im einfachsten Fall als flache Kupferringe ausgebildet. Das von der Bestrahlungsquelle 2 emittierte gepulste Licht 20 wird von dem Talgpropf 21 und dem darunterliegenden Talg 22 absorbiert. Dabei absorbiert der Talgpropf 21 im sichtbaren Spektralbereich und im NIR, wohingegen der Talg 22 vorzugsweise im NIR abscrbiert. Hierdurch kommt es zu einer Erwärmung und Verflüssigung des Talgpropfes 21 bzw. Talgs 22. Durch geeignete Polung der Flachspulen 18, 19 kommt es zu einer Anziehung bzw. Abstossung zwischen den Flachspulen 18, 19. Da der Rahmen 15 fest ist, bewegt sich daher der Stempel 16 auf die Haut zu oder von dieser weg, was durch den Doppelpfeil 23 verdeutlicht wird. Durch diese Rüttelbewegung wird der verflüssigte Talgpropf 21 gelöst und der Talgpropf 21 und der Talg 22 werden herausgesaugt.

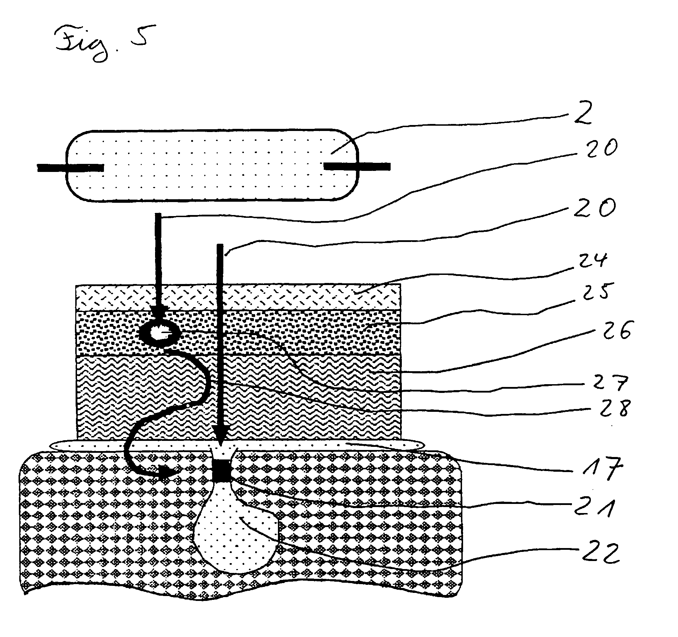

In der Fig.5 ist eine alternative Ausführungsform zur Erzeugung von mechanischen Schwingungen dargestellt. Die Einrichtung umfaßt eine erste Schicht 24 aus einem optisch transparentem Material mit hoher Schall-Leitungsgeschwindigkeit, eine zweite Schicht 25 aus einem optisch transparentem Trägermaterial und einer dritten Schicht 26. Auf und/oder in der zweiten Schicht sind lichtabsorbierende Farbstoffmoleküle 27 angeordnet, die beispielsweise streifen- oder ringförmig angeordnet sind. Aufgrund der Absorption von einfallendem gepulsten Licht 20, kommt es zu einer schockartigen thermischen Expansion der Farbstoffmoleküle 27, wodurch eine Druckwelle 28 aufgebaut wird. Diese Druckwelle 28 ist ungerichtet und breitet sich nach oben und unten aus. Die Druckwelle 28, die sich nach oben ausbreitet wird dabei an der ersten Schicht 24 reflektiert und wieder nach unten abgestrahlt. Die dritte Schicht 26 dient hingegen zur einer gezielten Laufzeitverschiebung von Licht und Druckwelle 28, sodass die Druckwelle 28 erst den Talgpropf 21 erreicht, nachdem der Lichtpuls diesen angewärmt und verflüssigt hat. Die dritte Schicht 26 kann auch entfallen, wenn gezielt Nahfeldeffekte ausgenutzt werden. Hierzu werden lokale Maxima erzeugt, die bei Frequenzen im kHz-Bereich näher als λ/2 an der Hautoberfläche liegen.

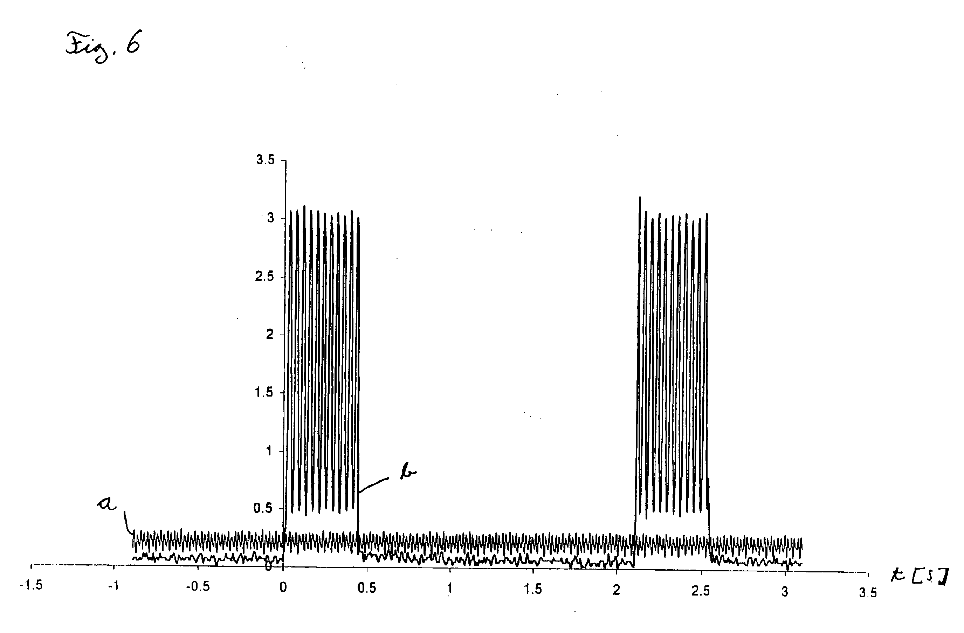

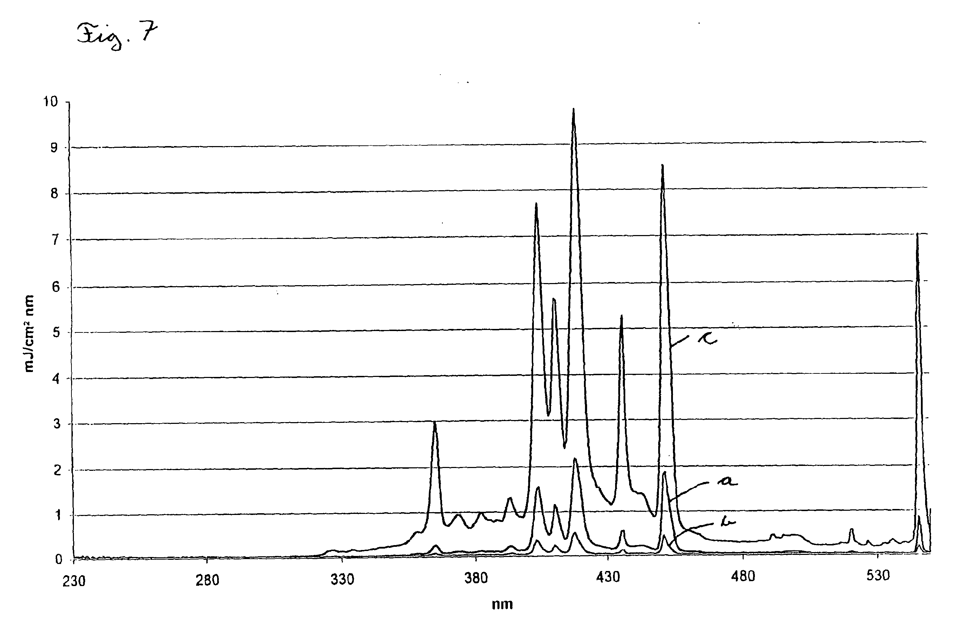

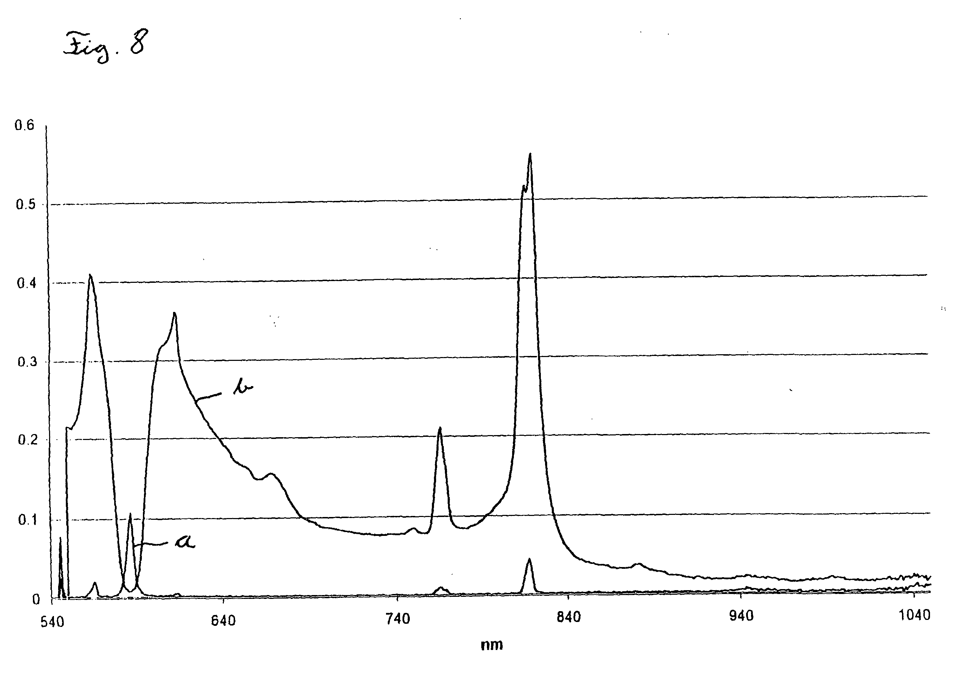

In der Fig.6 ist die relative Bestrahlungsstärke einer 1000 W Quecksilberiodid-Lampe im cw-Normalbetrieb bei 1000 W (Verlauf a) und im Pulsüberlastbetrieb (Verlauf b) dargestellt, wobei die gemittelte cw-Leistung im Pulsbetrieb 1500 W beträgt. Wie deutlich erkennbar, kommt es bereits bei geringer Überlast zu einer erheblichen Erhöhung der Emission. In der Fig.7 ist die spektrale Energiedichte bei einer derartigen galliumdotierten Quecksilberiodid-Lampe mit Nennleistung 1000 W dargestellt, wenn die Eingangsleistung variiert wird. Dabei stellt der Verlauf a) die Energiedichte im cw-Normalbetrieb bei 1000 W dar. Der Verlauf b) stellt die spektrale Energiedichte bei Unterlast von 100 W und der Verlauf c) stellt die Energiedichte bei 10 kW-Eingangsleistung dar, wobei hierbei sowohl Unter- als auch Überlast im cw-Betrieb betrieben wurden. Wie man jedoch deutlich erkennt, bleiben in beiden Fällen die Spektrallinien erhalten und es findet keine Inversion der Spektrallinien statt, sondern es kommt zu einer nahezu proportionalen Erhöhung der Emission. Im Gegensatz hierzu erkennt man deutlich in Fig.8 in Verlauf b) die Inversion der Spektrallinien bei einer Natriumdampflampe, die im Pulsbetrieb mit 700 W bei 230 W Nennleistung betrieben wird. Im Vergleich hierzu ist im Verlauf a) die relative Bestrahlungsstärke bei cw-Nennbetrieb dargestellt.

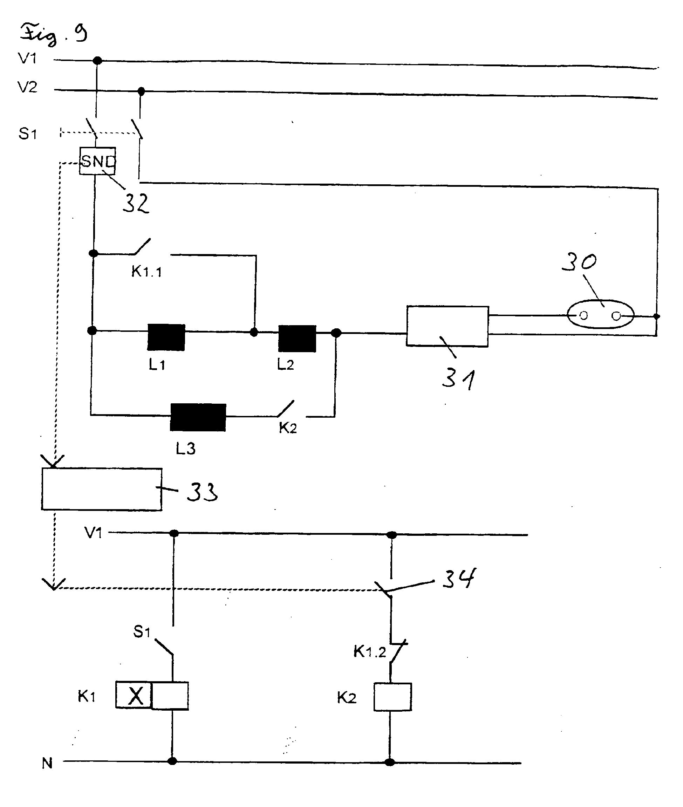

In der Fig.9 ist eine erste Schaltungsanordnung zum Betrieb einer galliumdotierten Quecksilberiodid-Lampe zum Pulsüberlastbetrieb dargestellt. Die Schaltung umfaßt eine galliumdotierte Quecksilberiodid-Lampe 30, einen Zündungsstab 31, einen Strom-Null-Durchgangsdetektor 32, einen Impulsgenerator 33, ein erstes Relais K1 und ein zweites Relais K2, sowie einen Starterschalter S1 und einen Impulsschalter 34. Die beiden Relais K1 und K2 sind beide mit einem Nullleiter N und einer ersten Phase V1 eines Drehstromnetzes verbunden. Die galliumdotierte Quecksilberiodid-Lampe 30 ist über einen Hilfskontakt des Starterschalters S1 mit einer zweiten Phase V2 des Drehstromnetzes verbunden. Über einen zweiten Hilfskontakt des Starterschalters S1 ist die erste Phase V1 über den Strom-Null-Durchgangsdetektor 32 über eine Spulenanordnung mit dem Zündungsstab 31 verbunden. Dabei sind die Spulen L1 und L2 in Reihe geschaltet. Parallel zu dieser Reihenschaltung ist eine dritte Spule L3 mit einem zum zweiten Relais K2 zugehörigen Kontakt K2 geschaltet. Parallel zur ersten Spule L1 ist ein erster zugehöriger Kontakt K1.1 des ersten Relais K1 geschaltet. Zwischen dem zweiten Relais K2 und dem impulsschalter 34 ist ein zweiter zugehöriger Kontakt K1.2 des ersten Relais K1 geschaltet. Die prinzipielle Funktionsweise der Schaltung ist folgende:
Zuerst wird der Starterschalter S1 geschlossen, wodurch auch die beiden zugeordneten Hilfskontakte schliessen. Dadurch schließt der Kontakt K1.1 und der Kontakt K1.2 öffnet bzw. bleibt offen. Über den geschlossenen Kontakt K1.1 ist die erste Phase V1 des Drehstromnetzes über die Spule L2 mit dem Zündungsstab 31 verbunden, wobei die Spule L2 als Vorschaltdrossel wirkt. Dieser Schaltzustand bleibt solange erhalten, bis die galliumdotierte Quecksilberiodid-Lampe 30 ihre normalen Betriebsbedingungen erreicht hat. Anschließend fällt das Relais K1 ab, das beispielsweise als Einschaltwischer ausgebildet ist. Der Abfall von dem Relais K1 bewirkt ein öffnen des Kontaktes K1.1 und ein schließen des Kontaktes K1.2. Hierdurch wird das Relais K2 aktiviert und gleichzeitig die Spule L1 in Reihe zur Spule L2 geschaltet, wobei die Spule L1 als Simmerspule wirkt. Da der der Impulsschalter 34 noch offen ist, bleibt der Kontakt K2 noch offen. In diesem Zustand läuft die galliumdotierte Quecksilberiodid-Lampe 30 in einem Simmer. Zum Pulsbetrieb erfaßt nun der Strom-Null-Durchgangsdetektor 32 einen Nulldurchgang des Stromes der ersten Phase V1 des Drehstromnetzes und signalisiert dies dem Impulsgenerator 33. Dieser schaltet den Impulsschalter 34, so daß das Relais K2 bestromt wird und der Kontakt K2 geschlossen wird. Hierdurch wird die Spule L3 parallel geschaltet und die Gesamtinduktivität sinkt, wodurch der Zündungsstab 31 einen Überlastimpuls erhält. Am Ende des Pulses öffnet der Impulsgenerator 33 im Stromnulldurchgang den Impulsschalter 34. Dadurch wird der Kontakt K2 geöffnet und die galliumdotierte Quecksilberiodid-Lampe 30 wird wieder im Simmer durch die Reihenschaltung der Spulen L1 und L2 betrieben, bis der nächste Impuls durch den Impulsgenerator 33 ausgelöst wird.

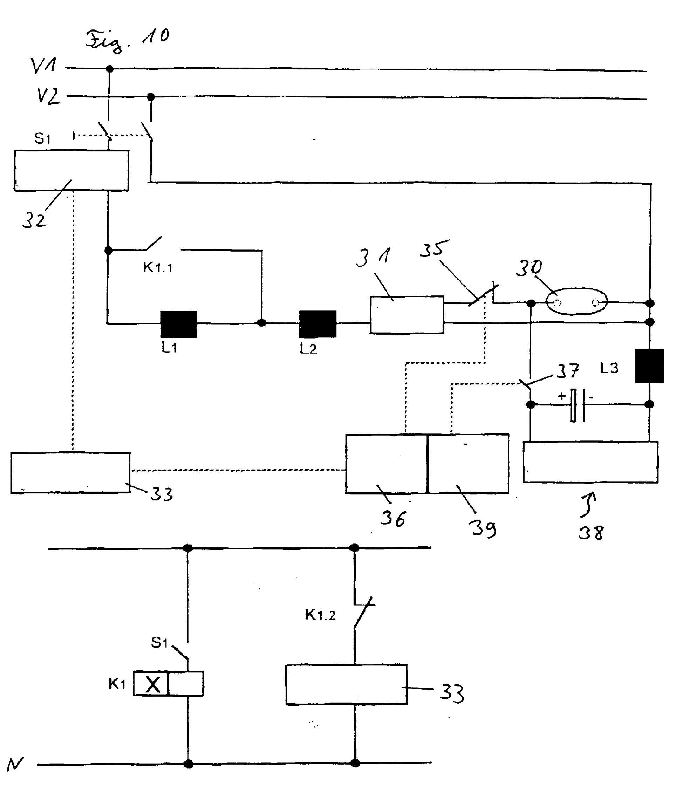

In der Fig.10 ist eine alternative Ausführungsform mit einer Kondensatorbank dargestellt, wobei gleiche Elemente in Bezug auf Fig.9 mit gleichen Bezugszeichen versehen sind. Im Unterschied zu der Ausführungsform gemäß Fig.9 ist zwischen dem Zündungsstab 31 und der galliumdotierten Quecksilberiodid-Lampe 30 ein Triac 35 angeordnet, dessen Treiber 36 vom Impulsgenerator 33 angesteuert wird. Über einen IGBT 37 bzw. die Spule L3 ist die Kondensatorbank 38 mit den Elektroden der galliumdotierten Quecksilberiodid-Lampe 30 verbunden, wobei der Treiber 39 des IGBT 37 ebenfalls von dem Impulsgenerator 33 angesteuert wird. Die Funktionsweise ist dabei wie folgt:
Wieder wird der Starterschalter S1 geschlossen, wodurch der Kontakt K1.1 schließt und der Kontakt K1.2 öffnet. Über den durchgeschalteten Triac 35 wird die galliumdotierte Quecksilberiodid-Lampe 30 auf Betriebsbedingungen hochgefahren. Anschließend fällt wieder das Relais K1 ab und der Kontakt K1.1 öffnet und K1.2 schließt. Die galliumdotierte Quecksilberiodid-Lampe 30 wird dann im Simmer über die Reihenschaltung der Spulen L1 und L2 betrieben und der Impulsgenerator 33 ist aktiviert. Für den Pulsbetrieb erfaßt dann der Strom-Null-Durchgangsdetektor 32 den Nulldurchgang und überträgt diese Information an den Impulsgenerator 33. Dieser steuert daraufhin die Treiber 36 und 39, so daß der Triac 35 sperrt und der IGBT 37 leitet. Dadurch wird die Kondensatorbank auf die galliumdotierte Quecksilberiodid-Lampe 30 geschaltet und diese von der Netzspannung getrennt. Am Ende des Pulses wird umgekehrt beim Stromnulldurchgang der IGBT 37 gesperrt und der Triac 35 durchgeschaltet., so daß die Lampe 30 wieder im Simmer über die beiden Spulen L1 und L2 läuft.
Dabei sei angemerkt, daß wenn zuvor von Spulen die Rede war hierunter allgemein Induktivitäten zu verstehen sind. Zur Veranschaulichung der Größenverhältnisse dienen folgende Werte für die Spulen L1-L3:
L1=500 mH; L2=150 mH und L3= 7 mH.

Dabei stellen sich folgende Werte für Strom bzw. Stromdichte ein, wobei weiter unten zum Vergleich die Werte für cw-Normalbetrieb angegeben sind:
Pulsbetrieb: I_{eff}=40 A bzw. 11,8 A/cm², Iₚₑₐₖ=55 A bzw. 16,2 A/cm²;
Simmerbetrieb:I_{eff}=1,2 A bzw. 0,35 A/cm², Iₚₑₐₖ=1,7 A bzw. 0,5 A/cm²;
Normalbetrieb: I_{eff}=5 A bzw.1,5 A/cm², Iₚₑₐₖ=7 A bzw. 2 A/cm²;

## Patentansprüche

1. Bestrahlungsanordnung zur Behandlung von Akne, Aknenarben und Hautalterungen, umfassend mindestens eine Bestrahlungsquelle,
**dadurch gekennzeichnet, daß**
die Bestrahlungsquelle (2) ein breitbandiges optisches Spektrum im Bereich von mindestens 320 bis mindestens 540 nm emittiert, die Bestrahlungsquelle (2) zur Erzeugung von optischen Pulsen im Pulsbetrieb betreibbar und/oder relativ zu der zu behandelnden Fläche durch einen Scanner bewegbar ist, wobei, wenn die Bestrahlungsanordnung auf die Oberfläche aufgesetzt würde, die Pulsenergie zwischen 0,05-10 J/cm² liegt und die Bestrahlungsstärke der Bestrahlungspeaks der optischen Pulse größer als 0,5 W/cm² und kleiner als 100 kW/cm² ist, der Anordnung eine Einrichtung zur Erzeugung einer mechanischen Schwingung zugeordnet ist, die auf die zu behandelnde Haut aufgesetzt wird.

2. Bestrahlungsanordnung nach Anspruch 1, **dadurch gekennzeichnet, daß** die Schwingungserzeugung zeitlich versetzt zur Abgabe der Pulse erfolgt.

3. Bestrahlungsanordnung nach Anspruch 1 oder 2, **dadurch gekennzeichnet, daß** die Einrichtung als elektrodynamischer oder photoelastischer Transducer ausgebildet ist.

4. Bestrahlungsanordnung nach einem der vorangegangen Ansprüche, **dadurch gekennzeichnet, daß** die effektive Pulslänge zwischen 1 µs - 500 ms liegt.

5. Bestrahlungsanordnung nach Anspruch 4, **dadurch gekennzeichnet, daß** die Bestrahlungsquelle (2) mit einer Frequenz von 0,01 - 100 Hz getaktet ist.

6. Bestrahlungsanordnung nach einem der vorangegangenen Ansprüche, **dadurch gekennzeichnet, daß** die Bestrahlungsquelle (2) als Xe- oder Deuterium-Blitzlampe oder als galliumdotierte Quecksilberiodid-Lampe ausgebildet ist, die mit Impulsüberlast betrieben wird.

7. Bestrahlungsanordnung nach einem der vorangegangenen Ansprüche, **dadurch gekennzeichnet, daß** der Bestrahlungsquelle (2) eine Einrichtung zur Unterdrückung der Spektralanteile von 320 - 400 nm und/oder zur Transformierung der UV-Anteile in den sichtbaren Bereich zugeordnet ist.

8. Bestrahlungsanordnung nach Anspruch 7, **dadurch gekennzeichnet, daß** vor der Bestrahlungsquelle (2) eine Leuchtstofffolie (8) angeordnet ist.

9. Bestrahlungsanordnung nach Anspruch 8, **dadurch gekennzeichnet, daß** die Leuchtstofffolie (8) aus einem Silikonelastomer besteht und mit anorganischen Leuchtstoffpartikeln dotiert ist.

10. Bestrahlungsanordnung nach Anspruch 8 oder 9, **dadurch gekennzeichnet, daß** die Leuchtstofffolie (8) mit mindestens einem der nachfolgenden Leuchtstoffpartikeln, fluoreszierend in den Spektralbereichen 370-460 nm [Sr₂ P₂ O₇ :Eu, Sr₅(PO₄)₃Cl:Eu, BaMg₂Al₁₆ O₂₇ :Eu, Ca WO₄ : Pb; (Sr, Ca, Ba)₅(PO₄)₃Cl:EU; Sr₂P₂O₇:Sn; (Ba, Ca)₅(PO₄)₃Cl:Eu)]
und/oder 510-560 nm
[ZnSlO₄:Mn; MgAl₁₁O₁₉:Ce,Tb,Mn; YBO₃:Tb; LaPO₄:Ce,Tb]
und/oder 610-670 nm
[Y₂O₃:EU; Y(P,V)O₄:Eu; CaSlO₃:Pb,Mn; (Sr,Mg)₃(PO₄)₂:Sn; 3.5MgO*0.5MgF₂*GeO₂:Mn ]
dotiert ist.

11. Bestrahlungsanordung nach einem der vorangegangenen Ansprüche, **dadurch gekennzeichnet, daß** der Bestrahlungsanordnung (1) Mittel zur topischen und/oder inspiratorischen Sauerstoffzufuhr zugeordnet sind.

12. Bestrahlungsanordnung nach einem der vorangegangenen Ansprüche, **dadurch gekennzeichnet, daß** der Bestrahlungsanordnung (1) eine Einrichtung zur Kühlung einer zu bestrahlenden Fläche (7) und/oder der Leuchtstofffolie (8) zugeordnet ist.

13. Bestrahlungsanordnung nach einem der vorangegangenen Ansprüche, **dadurch gekennzeichnet, daß** die Einrichtung als Luft- oder Wasserkühlung ausgebildet ist.

## Claims

1. Irradiation arrangement for the treatment of acne, flaws caused by acne and skin-ageing phenomena, said arrangement including at least one source of irradiation, **characterised in that** the source of irradiation (2) emits a wide-band optical spectrum in the range of at least 320 to at least 540 nm; the source of irradiation (2) for the generation of optical pulses is operable in the pulse operation and/or is displaceable relative to the surface to be treated by means of a scanner, the pulse energy being between 0.05 and 10 J/cm², and the irradiation intensity of the irradiation peak of the optical pulses being greater than 0.5 W/cm² and smaller than 100 kW/cm², if the irradiation arrangement were placed upon the surface, and an apparatus for generating a mechanical oscillation being associated with the arrangement, which apparatus is placed upon the skin to be treated.

2. Irradiation arrangement according to claim 1, **characterised in that** the oscillation is generated in a staggered manner with respect to time in order to release the pulses.

3. Irradiation arrangement according to claim 1 or 2, **characterised in that** the apparatus is in the form of an electrodynamic or a photoelastic transducer.

4. Irradiation arrangement according to one of the preceding claims, **characterised in that** the effective pulse length is between 1 µs and 500 ms.

5. Irradiation arrangement according to claim 4, **characterised in that** the source of irradiation (2) is set by a frequency of 0.01 to 100 Hz.

6. Irradiation arrangement according to one of the preceding claims, **characterised in that** the source of irradiation (2) is in the form of a Xe flashlight or a deuterium flashlight or a gallium-doped mercury iodide light, which is operated with pulse overloading.

7. Irradiation arrangement according to one of the preceding claims, **characterised in that** an apparatus for suppressing the spectral proportions of 320 to 400 nm and/or for transforming the UV proportions into the visible range is associated with the source of irradiation (2).

8. Irradiation arrangement according to claim 7, **characterised in that** a luminous film (8) is disposed in front of the source of irradiation (2).

9. Irradiation arrangement according to claim 8, **characterised in that** the luminous film (8) is formed from a silicone elastomeric material and is doped with inorganic luminous particles.

10. Irradiation arrangement according to claim 8 or 9, **characterised in that** the luminous film (8) is doped with at least one of the following luminous particles, fluorescent in the spectral ranges 370 to 460 nm [Sr₂ P₂ O₇ :Eu, Sr₅(PO₄)₃Cl:Eu, BaMg₂Al₁₆ O₂₇ :Eu, Ca WO₄ : Pb; (Sr, Ca, Ba)₅(PO₄)₃Cl:Eu; Sr₂P₂O₇:Sn; (Ba, Ca)₅(PO₄)₃Cl:Eu)]
and/or 510 to 560nm
[ZnSIO₄:Mn; MgAl₁₁ O₁₉:Ce, Tb,Mn; YBO₃:Tb; LaPO₄:Ce,Tb]
and or 610 to 670 nm
[Y₂O₃:Eu; Y(P,V)O₄:Eu; CaSIO₃:Pb,Mn; (Sr,Mg)₃(PO₄)₂:Sn;
3.5MgO*0.5MgF₂*GeO₂:Mn].

11. Irradiation arrangement according to one of the preceding claims, **characterised in that** means for the topical and/or inspiratoric supply of oxygen are associated with the irradiation arrangement (1).

12. Irradiation arrangement according to one of the preceding claims, **characterised in that** an apparatus for cooling a surface (7) to be irradiated and/or the luminous film (8) is associated with the irradiation arrangement (1).

13. Irradiation arrangement according to one of the preceding claims, **characterised in that** the apparatus is in the form of an air- or water-cooling means.

## Revendications

1. Dispositif d'irradiation en vue du traitement de l'acné, de cicatrices provenant d'acné et de vieillissements de la peau, comportant au moins une source de rayonnement, **caractérisé en ce que**,
la source de rayonnement (2) émet un spectre optique à bande large dans le domaine d'au moins 320 à au moins 540 nm, **en ce que** la source de rayonnement (2) peut être opérée en un mode pulsé en vue de la production d'impulsions optiques et/ou peut être déplacée par rapport à la surface à traiter à l'aide d'un dispositif de balayage, l'énergie des impulsions se situant entre 0,05-10 J/cm² et l'intensité de rayonnement des pics de rayonnement des impulsions optiques étant supérieure à 0,5 W/cm² et inférieure à 100 kW/cm², lorsque le dispositif d'irradiation a été placé sur la surface, un équipement en vue de la production d'une oscillation mécanique, qui est placé sur la peau à traiter, étant affecté au dispositif.

2. Dispositif d'irradiation selon la revendication 1, **caractérisé en ce que** la production d'oscillations se fait avec un décalage temporel par rapport à l'émission des impulsions.

3. Dispositif d'irradiation selon la revendication 1 ou 2, **caractérisé en ce que** l'équipement est réalisé en tant qu'un transducteur électrodynamique ou photoélastique.

4. Dispositif d'irradiation selon l'une quelconque des revendications précédentes, **caractérisé en ce que** la longueur d'impulsion effective se situe entre 1 µs et 500 ms.

5. Dispositif d'irradiation selon la revendication 4, **caractérisé en ce que** la source de rayonnement (2) est synchronisée à une fréquence de 0,01 -100 Hz.

6. Dispositif d'irradiation selon l'une quelconque des revendications précédentes, **caractérisé en ce que** la source de rayonnement (2) est réalisée en tant qu'une lampe flash au Xe ou au deutérium ou en tant qu'une lampe à iodure de mercure dopée au gallium, qui est opérée en mode de surcharge d'impulsions.

7. Dispositif d'irradiation selon l'une quelconque des revendications précédentes, **caractérisé en ce que** l'on associe à la source de rayonnement (2) un équipement en vue de la suppression des portions spectrales de 320 - 400 nm et/ou en vue de la transformation des portions UV dans le domaine visible.

8. Dispositif d'irradiation selon la revendication 7, **caractérisé en ce que** l'on dispose, avant la source de rayonnement (2), une feuille à substances luminescentes (8).

9. Dispositif d'irradiation selon la revendication 8, **caractérisé en ce que** la feuille à substances luminescentes (8) se compose d'un élastomère de silicones et est dopée à l'aide de particules de substances luminescentes inorganiques.

10. Dispositif selon la revendication 8 ou 9, **caractérisé en ce que** la feuille à substances luminescentes (8) est dopé à l'aide d'au moins un des particules de substances luminescentes suivantes, émettant une fluorescence dans les domaines spectraux 370-460 nm
[Sr₂P₂O₇ :Eu, Sr₅(PO₄)₃Cl:Eu, BaMg₂Al₁₆O₂₇ :Eu, Ca WO₄ : Pb ; (Sr, Ca,
Ba)₅(PO₄)₃Cl:Eu; Sr₂P₂0₇:Sn; (Ba, Ca)₅(PO₄)₃Cl:Eu)]
et/ ou 510-560 nm
[ZnSiO₄:Mn; MgAl₁₁O₁₉:Ce,Tb,Mn ; YBO₃:Tb; LaPO₄:Ce,Tb]
et/ou 610-670 nm
[Y₂O₃:Eu; Y(P,V)O₄:Eu; CaSiO₃:Pb,Mn; (Sr,Mg)₃(PO₄)₂:Sn ;
3,5MgO*0,5MgF₂*GeO₂:Mn].

11. Dispositif d'irradiation selon l'une quelconque des revendications précédentes, **caractérisé en ce que** l'on associe au dispositif d'irradiation (1) des moyens en vue de l'apport d'oxygène topique et/ ou inspiratoire.

12. Dispositif d'irradiation selon l'une quelconque des revendications précédentes, **caractérisé en ce que** l'on associe au dispositif d'irradiation (1) un équipement en vue du refroidissement d'une surface à irradier (7) et/ou de la feuille à substances luminescentes (8).

13. Dispositif d'irradiation selon l'une quelconque des revendications précédentes, **caractérisé en ce que** l'équipement est réalisé en tant qu'un dispositif de refroidissement à l'air ou à l'eau.
